## Europäisches Patentamt

## European Patent Office

## Office européen des brevets

(19)

(11) Veröffentlichungsnummer: **0 103 735 B1**

(12) **EUROPÄISCHE PATENTSCHRIFT**

(45) Veröffentlichungstag der Patentschrift:
25.09.85

(51) Int. Cl.⁴: **C 07 C 51/54**, C 07 C 51/56, C 07 C 53/12

(21) Anmeldenummer: **83107927.2**

(22) Anmeldetag: **11.08.83**

(54) **Verfahren zur Herstellung von Essigsäureanhydrid.**

(30) Priorität: **21.08.82 DE 3231154**

(43) Veröffentlichungstag der Anmeldung:
**28.03.84 Patentblatt 84/13**

(45) Bekanntmachung des Hinweises auf die Patenterteilung:
**25.09.85 Patentblatt 85/39**

(84) Benannte Vertragsstaaten:
**BE DE FR GB IT NL**

(56) Entgegenhaltungen:
**EP - A - 0 055 622**
**EP - A - 0 070 180**
**DE - A - 879 988**
**FR - A - 2 517 297**

(73) Patentinhaber: **HOECHST AKTIENGESELLSCHAFT,
Postfach 80 03 20, D-6230 Frankfurt am Main 80 (DE)**

(72) Erfinder: **Vogt, Wilhelm, Dr., Bellerstrasse 74,
D-5030 Hürth (DE)**
Erfinder: **Glaser, Hermann, Magdalenenweg 16,
D-5042 Erftstadt (DE)**
Erfinder: **Jägers, Erhard, Dr., Schwarzwaldstrasse 1,
D-5303 Bornheim (DE)**

## Beschreibung

Die Erfindung betrifft ein Verfahren zur Herstellung von Essigsäureanhydrid durch Umsetzung von Methylacetat und/oder Dimethylether mit Kohlenmonoxid, ggf. in Mischungen mit bis zu 20 Vol% Wasserstoff, unter praktisch wasserfreien Bedingungen bei Temperaturen von 120 bis 270° C und Drücken von 1-500 bar in Gegenwart eines Katalysatorsystems aus Nickel oder Nickelverbindungen, einer organischen Jod- oder Bromverbindung sowie einer tertiären oder quaternären organischen Phosphorverbindung.

Ein derartiges Verfahren kann bereits der US-PS Nr. 2729651 entnommen werden. Während dort überwiegend Nickel-Komplexe wie z.B. Triphenyl-ethyl-phosphoniumnickeltetrajodid eingesetzt werden, kann auch von z.B. Tetramethylammoniumjodid und Nickeljodid oder von Nickelpulver, Jod, Triethylamin und Ethyljodid ausgegangen werden. Obwohl Drücke bis zu 700 bar angewendet werden, sind die Raum-Zeit-Leistungen an Essigsäureanhydrid nur gering. Die Umsetzungszeiten liegen zwischen 5 und 26 Stunden.

Da das Reaktionsmedium korrosiv ist, so dass der Autoklav aus einer Legierung von Hastelloy B oder C oder aus Tantal hergestellt werden muss, bedürfen derartige Carbonylierungsverfahren eines sehr hohen Investitionsaufwandes.

Die vorliegende Erfindung ist nun dadurch gekennzeichnet, dass das Katalysatorsystem zusätzlich eine Titan- oder Zirkoniumverbindung enthält.

Dadurch wird eine Aktivierung des in der US-PS Nr. 2729651 beschriebenen Katalysatorsystems erreicht, wodurch erheblich höhere Raum-Zeit-Leistungen erhalten werden, was wiederum die Wirtschaftlichkeit des Verfahrens entscheidend verbessert.

Die ältere europäische Patentanmeldung Nr. 0070180 A1 offenbart ein Verfahren zur Herstellung von Essigsäureanhydrid durch Carbonylierung von Methylacetat oder Dimethylether in Gegenwart eines Flüssigphase-Katalysators, welcher Nickel oder dessen Verbindungen, Methyljodid und Verbindungen eines Metalls aus der 4. Hauptgruppe, vorzugsweise des Zinns, zusammen mit einer Verbindung eines Metalls aus der 1. oder der 2. Hauptgruppe des Periodensystems der Elemente enthält. Neben diesen zwingend erforderlichen Verbindungen kann der Katalysator zusätzlich noch Verbindungen des Titans oder des Zirkons enthalten. Die Anwesenheit organischer Phosphorverbindungen ist nicht vorgesehen.

Vorzugsweise setzt man im Verfahren der Erfindung Oxyalkylverbindungen des Titans oder Zirkoniums ein, z.B. Titansäureester wie Tetrabutyltitanat, Tetramethyltitanat; Titanylacetylacetonat oder Zirkonsäureester wie Tetrabutylzirkonat, ferner Zirkoniumacetylacetonat.

Als Nickelverbindungen kann man z.B. Nickelcarbonyl, Nickelacetylacetonat, Nickelhalogenid, Nickelacetat, Nickelsulfat oder Nickelcyanid einsetzen.

Als organische Jod- oder Bromverbindung eignen sich besonders Methyljodid, Ethyljodid, Methylbromid oder Ethylbromid. Als tertiäre oder quaternäre organische Phosphorverbindung verwendet man bevorzugt Trialkyl- oder Triarylphosphine bzw. deren Phosphoniumbromide oder -jodide, z.B. Tributylphosphin, Trioctylphosphin, Trilaurylphosphin, Triphenylphosphin, Tributylmethyl-phosphoniumjodid(-bromid), Trioctylmethyl-phosphoniumjodid(-bromid), Trilaurylmethyl-phosphoniumjodid(-bromid) oder Triphenyl-methyl-phosphoniumjodid(-bromid).

Man arbeitet bevorzugt bei Drücken von 10-300 bar. Die einzelnen Reaktionskomponenten, d.h. Methylacetat oder Dimethylether/Nickel(verbindung)/Jod- oder Bromverbindung/Phosphorverbindung/Titan- oder Zirkoniumverbindung, werden bevorzugt im Molverhältnis 1:(0,001-0,1):(0,01-1):(0,005-1):(0,0005-0,1) eingesetzt.

Durch Zusatz von z.B. 15 g Tetrabutyltitanat je Liter Reaktionslösung wurde bei 200° C eine Steigerung der Reaktionsgeschwindigkeit zu Essigsäureanhydrid um den Faktor 10 bis 15 beobachtet, ohne dass wesentliche Mengen Nebenprodukte aufgetreten wären.

Für das Katalysatorsystem mag kennzeichnend sein, dass Nickel beispielsweise in Form von Nickeltetracarbonyl bei Drücken unterhalb 200 bar und bei 200° C weniger gut zu beschleunigen vermag, wenn einer der wesentlichen Katalysatorbestandteile, zum Beispiel das Alkylhalogenid oder das Phosphin bzw. Phosphoniumsalz oder die Titan- bzw. Zirkoniumverbindung, weggelassen wird. Es ist im Sinne der vorliegenden Erfindung auch nicht möglich, das Phosphin gegen ein Alkyl- oder Arylamin auszuwechseln ohne starken Abfall der Raum-Zeit-Leistung an Essigsäureanhydrid. Bei Anwendung aller Katalysatorbestandteile im Umfange der Erfindung werden Raum-Zeit-Leistungen an Essigsäureanhydrid bei ≤ 200 bar Maximaldruck CO und ca. 200° C Reaktionstemperatur im Bereich von 500 bis 2000 g/l.h erreicht.

Durch Erhöhung der Katalysatorkonzentration und der Temperatur über 200° C hinaus lässt sich die Raum-Zeit-Leistung weiterhin steigern. Bei 230° C wurden beispielsweise 6000 g $Ac_2O$/l.h ermittelt, so dass dieses Verfahren für die Ausführung in kleinvolumigen Strömungsreaktionen prädestiniert erscheint.

Die erfindungsgemässe Carbonylierungsreaktion kann auch in Gegenwart von Lösemitteln durchgeführt werden. Als solche sind Essigsäure oder Amide wie N-Methylpyrrolidon N,N-Diethylacetamid oder schwefelhaltige Lösemittel wie Sulfolan besonders geeignet.

Es ist möglich, als Reaktionsgas eine Mischung von CO und $H_2$ bis zu 20 Vol% $H_2$-Gehalt anzuwenden, wenn für die Reaktion ein Kohlenmonoxid geringeren Reinheitsgrades angewendet werden soll. Es hat sich sogar gezeigt, dass die Aktivität wiederverwendeter Katalysatorlösungen weniger beeinträchtigt wird, wenn das Reaktionsgas 2-20 Vol% Wasserstoff enthält.

*Beispiele 1 bis 5 (Vergleichsbeispiele)*

In einem korrosionsfesten Edelstahlrührautoklaven von 1 Liter Inhalt wurden 250 g Methylacetat, 50 g Methyljodid und 60 g Methyl-tributylphosphoniumjodid eingefüllt. Nach Zugabe von jeweils 5 g Nickelkomponente in Form von

1. Nickelpulver
2. Nickelchlorid
3. Nickelacetat
4. Nickeltetracarbonyl
5. Nickelacetylacetonat

wurden nach gründlichem Spülen des Autoklaven mit Argon zur Entfernung von $O_2$ 100 bar CO auf- gedrückt. Der Autoklav wurde anschliessend auf 197° C erhitzt. Mit den angegebenen Nickelkom- ponenten wurden nachfolgende Ergebnisse erhal- ten. Aus Vergleichsgründen wird die Menge Essigsäureanhydrid angegeben, die während 1 Stunde aus 1 Liter Reaktionslösung entstanden war:

| Katalysator Ni-Verbindung | Gebildete Menge $(CH_3CO)_2O$ in g/l.h (Raum-Zeit-Leistung) |
|---|---|
| Ni-Pulver | 100 |
| $NiCl_2$ | 80 |
| $Ni(OOCCH_3)_2 \cdot 4 H_2O$ | 143 |
| $Ni(CO)_4$ | 126 |
| $Ni(C_5H_7O_2)_2$ | 106 |

Die Raum-Zeit-Leistungen konnten unter den gleichen Bedingungen und bei gleichen Einsatzmengen durch Eindrücken von 90 bar CO und 10 bar $H_2$ beträchtlich erhöht werden:

| Katalysator Ni-Verbindung | Gebildete Menge $(CH_3CO)_2O$ in g/l.h (Raum-Zeit-Leistung) |
|---|---|
| Ni-Pulver | 221 |
| $NiCl_2$ | 150 |
| $Ni(OOCCH_3)_2 \cdot 4 H_2O$ | 270 |
| $Ni(CO)_4$ | 400 |
| $Ni(C_5H_7O_2)_2$ | 320 |
| Nebenprodukte wie Ethylidendiacetat waren nur in Spuren nachweisbar. | |

*Beispiel 6*

In den Autoklaven wurden 250 g (3,38 Mol) Methylacetat, 50 g (0,352 Mol) Methyljodid, 60 g (0,174 Mol) $[CH_3(C_4H_9)_3P]$ J, 5 g (0,03 Mol) $Ni(CO)_4$, 5 g (0,015 Mol) $Ti(OC_4H_9)_4$ eingefüllt. Das Volumen der Reaktionslösung betrug 351 ml. Nach Spülen des Autoklaven und Aufpressen von 100 bar CO wurde auf 197° C erhitzt. Innerhalb von 16 min war der Druck im Autoklaven von 165 auf 75 bar abgefallen, wobei die Temperatur infolge der Reaktionswärme um 10° C anstieg. Nach Aufarbeitung der tiefroten Lösung durch Destilla- tion konnten 160 g Essigsäureanhydrid gewonnen

werden, entsprechend einer Leistung von 1710 g Essigsäureanhydrid je Liter Reaktionslösung und Stunde.

*Beispiel 7*

Die Einsatzmengen und die Reaktionszeit waren wie in Beispiel 6. Die 5 g Tetrabutyltitanat wurden durch 5 g (0,03 Mol) Tetramethyltitanat ersetzt. Die erhaltene Essigsäureanhydridmenge wurde nach destillativer Aufarbeitung und gas-chromatographischer Analyse mit 165 g bestimmt, entsprechend einer Leistung von 1753 g $(CH_3CO)_2O$ je Liter Reaktionslösung und Stunde.

*Beispiel 8*

Die Einsatzmengen waren wie in Beispiel 6. Das verwendete Tetrabutyltitanat wurde durch 5 g (0,01 Mol) Zirkoniumacetylacetonat ersetzt. Unter den gleichen Reaktions- und Aufarbeitungsbedingungen wurden in 19 min 161 g Essigsäurean- hydrid entsprechend 1450 g $(CH_3CO)_2O$ je Liter Reaktionslösung und Stunde erhalten.

*Beispiel 9*

Die Bedingungen waren die gleichen wie in Beispiel 6. Das Nickelcarbonyl wurde durch 5 g (0,02 Mol) Nickelacetylacetonat ersetzt. In 19 min wurden 161 g Essigsäureanhydrid entsprechend 1450 g $(CH_3CO)_2O$ je Liter Reaktionslösung und Stunde erhalten.

*Beispiel 10*

Einsatzmengen: 250 g Methylacetat, 50 g Methyljodid, 7,7 g (0,03 Mol) Nickelacetylacetonat, 11,5 g (0,03 Mol) Butylzirkonat, 60 g Methyltributylphosphoniumjodid. Aufgdrückt wurden 90 bar CO und 10 bar $H_2$. Bei 197° C konnten in 17 min 159 g Essigsäureanhydrid entsprechend 1600 g Essigsäureanhydrid je Liter Reaktionslösung und Stunde erhalten werden.

Die Reaktionstemperatur konnte infolge der ho- hen Reaktionsgeschwindigkeit nicht genau ein- gehalten werden und überschritt den Sollwert um bis zu 10° C.

*Beispiel 11*

Man arbeitete wie in Beispiel 10 und drückte 100 bar CO ohne Wasserstoff auf. In 18 min wurden 161 g Essigsäureanhydrid entsprechend 1530 g je Liter Reaktionsvolumen und Stunde erhalten.

*Beispiel 12*

Die bei der destillativen Aufarbeitung im Beispiel 6 erhaltenen Vorläufe aus Methyljodid und nicht umgesetztem Methylacetat wurden nach Zugabe von frischem Methylacetat mit dem Destil- lationsrückstand erneut eingesetzt. Nach insge- samt 5 Rückführungen war kein Leistungsabfall zu beobachten. Das aufgepresste CO-Gas enthielt 10 Vol% Wasserstoff. Die durchschnittlichen Leistungen lagen bei 1600 g $(CH_3CO)_2O$ je Liter Re- aktionslösung und Stunde.

*Beispiel 13*

In den 1-Liter-Autoklaven wurden
120 g $CH_3$ (0,84 Mol),
25 g $Ni(CO)_4$ (0,146 Mol),
150 g $CH_3(C_4H_9)_3P$ J (0,435 Mol),
5 g $Ti(OCH_3)_4$ (0,03 Mol

eingefüllt. Nach dem Eindrücken von 100 g Dimethylether (2,17 Mol) wurden 90 bar CO aufgedrückt. Nach dem Hochheizen auf 185° C stellte sich ein Maximaldruck von 155 bar ein. Innerhalb von 10 Minuten war der Reaktionsdruck auf 90 bar gefallen. Anschliessend wurde noch dreimal CO nachgedrückt.

Die gesamte CO-Aufnahme entsprach einer Druckverminderung von 160 bar und erforderte 1 Stunde. Nach Aufarbeitung konnten 156 g Essigsäureanhydrid und 16 g Methylacetat gewonnen werden.

**Patentansprüche**

1. Verfahren zur Herstellung von Essigsäureanhydrid durch Umsetzung von Methylacetat und/oder Dimethylether mit Kohlenmonoxid, ggf. in Mischungen mit bis zu 20 Vol% Wasserstoff, unter praktisch wasserfreien Bedingungen bei Temperaturen von 120 bis 270° C und Drücken von 1-500 bar in Gegenwart eines Katalysatorsystems aus Nickel oder Nickelverbindungen, einer organischen Jod- oder Bromverbindung sowie einer tertiären oder quaternären organischen Phosphorverbindung, dadurch gekennzeichnet, dass das Katalysatorsystem zusätzlich eine Titan- oder Zirkoniumverbindung enthält.

2. Verfahren nach Anspruch 1, dadurch gekennzeichnet, dass das Katalysatorsystem Oxyalkylverbindungen des Titans oder Zirkoniums enthält.

3. Verfahren nach Anspruch 1 oder 2, dadurch gekennzeichnet, dass man Methylacetat oder Dimethylether / Nickel(verbindung) / Jod- oder Bromverbindung/Phosphorverbindung/Titan- oder Zirkoniumverbindung im Molverhältnis 1 : (0,001-0,1) : (0,01-1) : (0,005-1) : (0,0005-0,1) einsetzt.

**Claims**

1. Process for making acetic anhydride by reacting methyl acetate and/or dimethylether with carbon monoxide, optionally in admixture with up to 20 volume % hydrogen, under practically anhydrous conditions at temperatures of 120 to 270° C, under pressures of 1 to 500 bars in the presence of a catalyst system containing nickel or a nickel compound, an organic iodine or bromine compound as well as a tertiary or quaternary organic phosphorus compound, wherein the catalyst system additionally contains a titanium or zirconium compound.

2. Process as claimed in Claim 1, wherein the catalyst system contains an oxyalkyl compound of titanium or zirconium.

3. Process as claimed in Claim 1 or 2, wherein methyl acetate or dimethylether/nickel(compound)/iodine or bromine compound/phosphorus compound/titanium or zirconium compound are used in a molar ratio of 1 : (0.001-0.1) : (0.01-1) : (0.0005-0.1).

**Revendications**

1. Procédé de préparation de l'anhydride acétique par réaction d'acétate de méthyle et/ou d'éther diméthylique avec le monoxyde de carbone, éventuellement en association avec jusqu'à 20% en volume d'hydrogène, dans des conditions pratiquement anhydres, à des températures de 120 à 270° C, sous des pressions de 1-500 bars, en présence d'un système catalytique constitué par du nickel ou des composés de nickel, un composé organique d'iode ou de brome ainsi qu'un composé organophosphoré tertiaire ou quaternaire, caractérisé en ce que le système catalytique contient en outre un composé de titane ou de zirconium.

2. Procédé selon la revendication 1, caractérisé en ce que le système catalytique contient des composés oxyalkyliques de titane ou de zirconium.

3. Procédé selon l'une des revendications 1 ou 2, caractérisé en ce que l'on utilise dans des proportions molaires les composants acétate de méthyle ou éther diméthylique/(composé de) nickel/composé d'iode ou de brome/composé de phosphore/composé de titane ou de zirconium de 1 : (0,001-0,1) : (0,01-1) : (0,005-1) : (0,0005-0,1).